(19) Europäisches Patentamt
European Patent Office
Office européen des brevets

(11) Veröffentlichungsnummer: **0 288 789 A1**

# (12) EUROPÄISCHE PATENTANMELDUNG

(21) Anmeldenummer: **88105492.8**

(22) Anmeldetag: **07.04.88**

(51) Int. Cl.4: **C07D 207/452 , C07D 209/48 , A01N 43/36 , C07C 131/00 , C07C 147/06 , C07C 147/14 , C07C 149/32**

(30) Priorität: **16.04.87 DE 3712987**

(43) Veröffentlichungstag der Anmeldung: **02.11.88 Patentblatt 88/44**

(84) Benannte Vertragsstaaten: **AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Stetter, Jörg, Dr.**
**Gellertweg 4**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Lürssen, Klaus, Dr.**
**August-Kierspel-Strasse 151**
**D-5060 Bergisch Gladbach 2(DE)**
Erfinder: **Strang, Harry, Dr.**
**Unterdorfstrasse 6A**
**D-4000 Duesseldorf 31(DE)**

(54) **N-Arylpyrrolin-2,5-dione.**

(57) N-Arylpyrrolin-2,5-dione der allgemeinen Formel

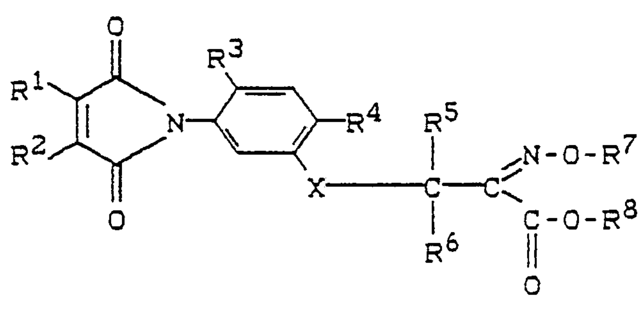

in welcher
$R^1$, $R^2$, $R^3$, $R^4$, $R^5$, $R^6$, $R^7$, $R^8$ und X die in der Beschreibung angegebene Bedeutung haben.
Verfahren zu ihrer Herstellung und ihre Verwendung als Herbizide und Wachstumsregulatoren.

EP 0 288 789 A1

## N-Arylpyrrolin-2,5-dione

Die Erfindung betrifft neue N-Arylpyrrolin-2,5-dione, mehrere Verfahren zu ihrer Herstellung und ihre Verwendung als Herbizide und Pflanzenwachstumsregulatoren.

Es ist bekannt, daß bestimmte N-Aryl-Stickstoffheterocylen, wie beispielsweise das N-(2,4-Dichlor-5-isopropoxyphenyl)-$\Delta^1$-tetrahydrophthalimid herbizide Eigenschaften besitzen (vgl. DE-OS 3 013 162).

Die herbizide Wirksamkeit dieser vorbekannten Verbindungen gegenüber Problemunkräutern ist jedoch ebenso wie ihre Verträglichkeit gegenüber bestimmten Kulturpflanzen nicht in allen Anwendungsgebieten völlig zufriedenstellend.

Über eine pflanzenwachstumsregulierende Wirkung der vorbekannten Verbindungen ist bisher nichts bekannt.

Es wurden neue N-Arylpyrrolin-2,5-dione der allgemeinen Formel (I),

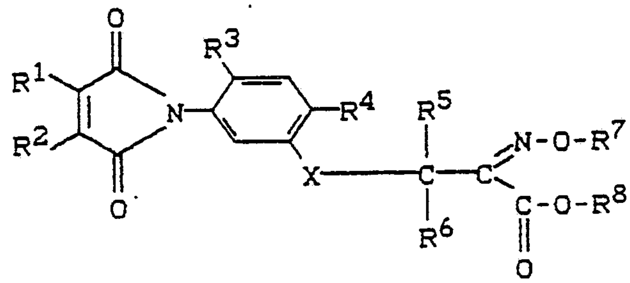

(I)

in welcher

$R^1$ und $R^2$ unabhängig voneinander jeweils für Wasserstoff, Alkyl, Halogenalkyl, Alkoxyalkyl, Alkoxycarbonylalkyl oder für gegebenenfalls substituiertes Aryl stehen, oder gemeinsam mit den Kohlenstoffatomen, an welche sie gebunden sind, für einen gegebenenfalls substituierten Cyclohexenylenrest oder für einen gegebenenfalls substituierten Phenylenrest stehen,

$R^3$ und $R^4$ unabhängig voneinander jeweils für Wasserstoff, Halogen, Alkyl, Alkoxy oder Alkylthio stehen,

$R^5$ für Wasserstoff, Alkyl oder für gegebenenfalls substituiertes Aryl steht,

$R^6$ für Wasserstoff oder Alkyl steht,

$R^7$ für Wasserstoff, Alkyl, Alkenyl, Alkinyl, Halogenalkyl, Halogenalkenyl, Alkoxyalkyl, Alkylthioalkyl, Alkoxycarbonylalkyl, Cyanalkyl, Cycloalkyl, Cycloalkylalkyl oder für gegebenenfalls substituiertes Aralkyl steht,

$R^8$ für Wasserstoff oder Alkyl steht und

X für Sauerstoff, Schwefel, eine Sulfinylgruppe oder eine Sulfonylgruppe steht,

gefunden.

Weiterhin wurde gefunden, daß man die neuen N-Arylpyrrolin-2,5-dione der allgemeinen Formel (I) nach einem der im folgenden beschriebenen Verfahren erhält:

(a) Man erhält N-Arylpyrrolin-2,5-dione der Formel (Ia),

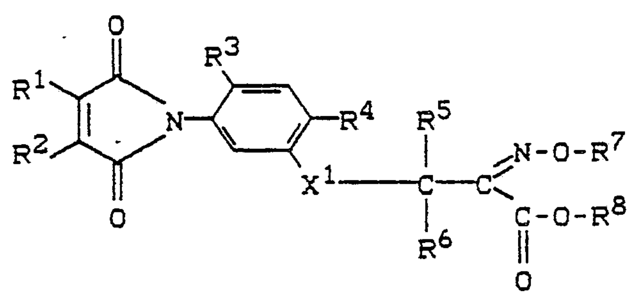

(Ia)

in welcher

$R^1$, $R^2$, $R^3$, $R^4$, $R^5$, $R^6$, $R^7$ und $R^8$ die oben angegebene Bedeutung haben und

$X^1$ für Sauerstoff oder Schwefel steht,

wenn man Pyrrolin-2,5-dione der Formel (II),

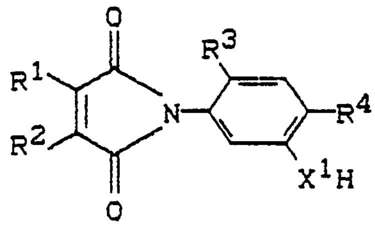

(II)

in welcher
$R^1$, $R^2$, $R^3$, $R^4$ und $X^1$ die oben angegebene Bedeutung haben,
mit α-Oximino-carbonsäurestern der Formel (III),

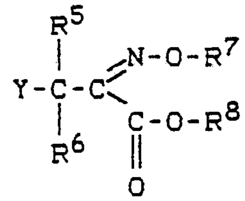

(III)

in welcher
$R^5$, $R^6$, $R^7$ und $R^8$ die oben angegebene Bedeutung haben und
Y für eine elektronenanziehende Abgangsgruppe steht,
gegebenenfalls in Gegenwart eines Verdünnungsmittels und gegebenenfalls in Gegenwart eines Säurebindemittels umsetzt;

(b) man erhält N-Arylpyrrolin-2,5-dione der Formel (Ib),

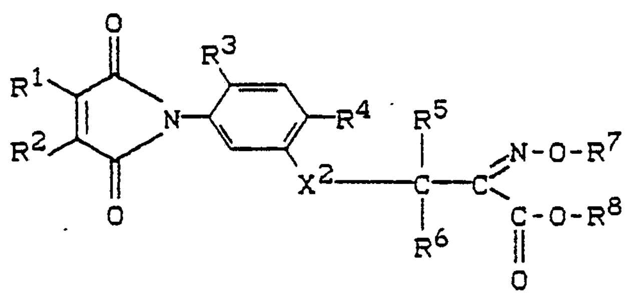

(Ib)

in welcher
$R^1$, $R^2$, $R^3$, $R^4$, $R^5$, $R^6$, $R^7$ und $R^8$ die oben angegebene Bedeutung haben und
$X^2$ für eine Sulfinyl oder eine Sulfonylgruppe steht,
wenn man die nach Verfahren (a) oder (c) erhältlichen N-Arylpyrrolin-2,5-dione der Formel (Ic),

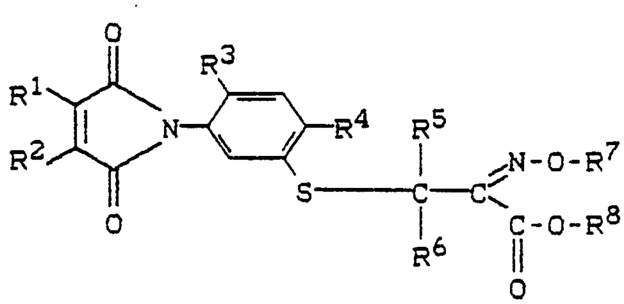

(Ic)

in welcher
$R^1$, $R^2$, $R^3$, $R^4$, $R^5$, $R^6$, $R^7$ und $R^8$ die oben angegebene Bedeutung haben,
mit einem Oxidationsmittel gegbenenfalls in Gegenwart eines Verdünnungsmittels, gegebenenfalls in Gegenwart eines Säurebindemittels und gegebenenfalls in Gegenwart eines Katalysators umsetzt;

(c) man erhält N-Arylpyrrolidin-2,5-dione der Formel (I),

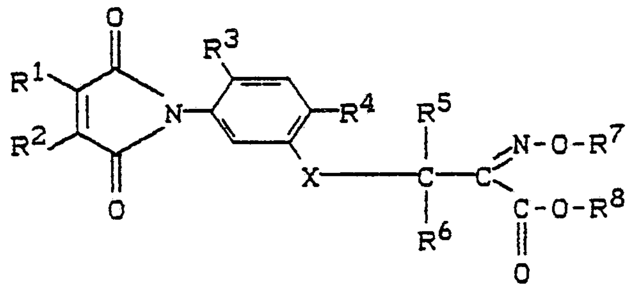

(I)

in welcher
$R^1$, $R^2$, $R^3$, $R^4$, $R^5$, $R^6$, $R^7$, $R^8$ und X die oben angegebene Bedeutung haben, alternativ auch, wenn man 1,2-Dicarbonsäureanhydride der Formel (IV),

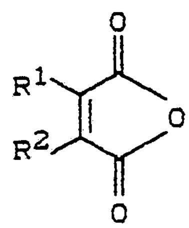

(IV)

in welcher
$R^1$ und $R^2$ die oben angegebene Bedeutung haben,
mit Anilinderivaten der Formel (V),

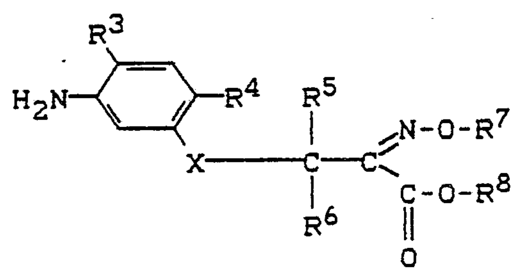

(V)

in welcher
$R^1$, $R^2$, $R^3$, $R^4$, $R^5$, $R^6$, $R^7$, $R^8$ und X die oben angegebene Bedeutung haben,
gegebenenfalls in Gegenwart eines Verdünnungsmittels und gegebenenfalls in Gegenwart eines Reaktionshilfsmittels umsetzt.

Schließlich wurde gefunden, daß die neuen N-Arylpyrrolin-2,5-dione der allgemeinen Formel (I) herbizide und wachstumstregulatorische Eigenschaften besitzen.

Überraschenderweise zeigen die erfindungsgemäßen N-Arylpyrrolin-2,5-dione der allgemeinen Formel (I) neben einer erheblich besseren herbiziden Wirksamkeit gegenüber wichtigen Problemunkräutern gleichzeitig eine deutlich verbesserte Verträglichkeit gegenüber wichtigen Kulturpflanzen im Vergleich zu den aus dem Stand der Tecknik bekannten N-Aryl-Stickstoffheterocyclen, wie beispielsweise das N-(2,4-Dichlor-5-isopropoxyphenyl)-$\Delta^1$-tetrahydrophthalimid, welches chemisch und wirkungsmäßig naheliegend Verbindungen sind und darüberhinaus unerwarteterweise zusätzliche eine pflanzenwachstumsregulierende Wirkung.

Die erfindungsgemäßen N-Arylpyrrolin-2;5-dione sind durch die Formel (I) allgemein definiert. Bevorzugt sind Verbindungen der Formel (I), bei welchen
$R^1$ und $R^2$ unabhängig voneinander jeweils für Wasserstoff, für jeweils geradkettiges oder verzweigtes Alkyl, Alkoxyalkyl oder Alkoxycarbonylalkyl mit jeweils 1 bis 4 Kohlen stoffatomen in einzelnen Alkylteilen, für geradkettiges oder verzweigtes Halogenalkyl mit 1 bis 4 Kohlenstoffatomen und 1 bis 9 gleichen oder verschiedenen Halogenatomen oder für gegebenenfalls einfach oder mehrfach, gleich oder verschieden substituiertes Aryl mit 6 bis 10 Kohlenstoffatomen stehen, wobei als Substituenten infrage kommen:

Halogen, Cyano, Nitro sowie jeweils geradkettiges oder verzweigtes Alkyl, Alkoxy, Alkylthio oder Halogenalkylmit jeweils 1 bis 4 Kohlenstoffatomen und im Fall des Halogenalkyl mit 1 bis 9 gleichen oder Verschiedenen Halogenatomen oder

$R^1$ und $R^2$ gemeinsam mit den Kohlenstoffatomen, an welche sie gebunden sind, für einen gegebenenfalls einfach oder mehrfach, gleich oder verschieden durch Alkyl mit 1 bis 4 Kohlenstoffatomen substituierten Cyclohexenylenrest oder für einen gegebenenfalls einfach oder mehrfach, gleich oder verschieden substituierten Phenylenrest stehen, wobei als Phenylen-Substituenten infrage kommen: Halogen, Cyano, Nitro sowie jeweils geradkettiges oder verzweigtes Alkyl, Alkoxy oder Alkoxycarbonyl mit jeweils 1 bis 4 Kohlenstoffatomen sowie Halogenalkyl mit 1 bis 4 Kohlenstoffatomen und 1 bis 9 gleichen oder verschiedenen Halogenatomen,

$R^3$ and $R^4$ unabhängig voneinander jeweils für Wasserstoff, Fluor, Chlor, Brom, Iod sowie jeweils geradkettiges oder verzweigtes Alkyl, Alkoxy oder Alkylthio mit jeweils 1 bis 4 Kohlenstoffatomen stehen,

$R^5$ für Wasserstoff, für geradkettiges oder verzweigtes Alkyl mit 1 bis 4 Kohlenstoffatomen oder für gegebenenfalls einfach oder mehrfach, gleich oder verschieden substituierts Aryl mit 6 bis 10 Kohlenstoffatomen steht, wobei als Substituenten infrage kommen: Fluor, Chlor, Brom, Iod, Cyano, Nitro sowie jeweils geradkettiges oder verzweigtes Alkyl oder Alkoxy mit jeweils 1 bis 4 Kohlenstoffatomen,

$R^6$ für Wasserstoff oder für geradkettiges oder verzweigtes Alkyl mit 1 bis 4 Kohlenstoffatomen steht,

$R^7$ für Wasserstoff, für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstofatomen, für jeweils geradketiges oder verzweigtes Halogenalkyl mit 1 bis 6 Kohlenstoffatomen oder Halogenalkenyl mit 3 bis 6 Kohlenstoffatomen und jeweils 1 bis 9 gleichen oder verschiedenen Halogenatomen, für jeweils geradkettiges oder verzweigtes Alkoxyalkyl, Alkylthioalkyl, Alkoxycarbonylalkyl oder Cyanalkyl mit jeweils 1 bis 4 Kohlenstoffatomen in den einzelnen Alkyl teilen, für Cycloalkyl mit 3 bis 7 Kohlenstoffatomen, für Cycloalkylalkyl mit 3 bis 7 Kohlenstoffatomen im Cycloalkylteil und 1 bis 4 Kohlenstoffatomen im geradkettigen oder verzweigten Alkylteil oder für gegebenenfalls im Arylteil einfach oder mehrach, gleich oder verschieden substituiertes, im Alkylteil geradkettiges oder verzweigtes Aralkyl mit 1 bis 4 Kohlenstoffatomen im Alkylteil und 6 bis 10 Kohlenstoffatomen im Arylteil steht, wobei als Arylsubstituenten infrage kommen: Halogen, Cyano, Nitro, sowie jeweils geradkettiges oder verzweigtes Alkyl, Alkoxy oder Alkylthio mit jeweils 1 bis 4 Kohlenstoffatomen,

$R^8$ für Wasserstoff oder für geradkettiges oder verzweigtes Alkyl mit 1 bis 4 Kohlenstoffatomen steht und

X für Sauerstoff, Schwefel, eine Sulfinylgruppe oder eine Sulfonylgruppe steht.

Besonders bevorzugt sind Verbindungen der Formel (I), bei welchen:

$R^1$ und $R^2$ unabhängig voneinander jeweils für Wasserstoff, Methyl, Ethyl, Isopropyl, Chlormethyl, Trifluormethyl, Methoxymethyl oder Methoxycarbonylmethyl stehen oder gemeinsam mit den Kohlenstoffatomen, an welche sie gebunden sind für einen gegebenenfalls ein-bis dreifach durch Methyl substituierten Cyclohexenylenrest oder für einen gegebenenfalls ein-bis vierfach, gleich oder verschieden durch Chlor, Nitro, Methyl und/oder Trifluormethyl substituierten Phenylenrest stehen,

$R^3$ und $R^4$ unabhängig voneinander jeweils für Wasserstoff, Fluor, Chlor, Brom, Methyl, Methoxy oder Methylthio stehen,

$R^5$ für Wasserstoff, Methyl, Ethyl, n-oder i-Propyl, n-, i-, s-oder t-Butyl oder für Phenyl steht,

$R^6$ für Wasserstoff, Methyl oder Ethyl steht,

$R^7$ für Wasserstoff, Methyl, Ethyl, n-oder i-Propyl, n-, i-, s-oder t-Butyl, Allyl, n-oder i-Butenyl, Propargly, n-oder i-Butinyl, für Chlorethyl, Trifluorethyl, Chlorallyl, Chlorbutenyl, Dichlorallyl, Cyclopropyl, Cyclopentyl, Cyclohexyl, Cyclopropylmethyl, Cyclopentylmethyl, Cyclohexylmethyl, für Methoxymethyl, Methoxyethyl, Ethoxymethyl, Ethoxyethyl, Methylthiomethyl, Methylthioethyl, Methoxycarbonylmethyl, Ethoxycarbonylmethyl, Methoxycarbonylethyl, Ethoxycarbonylethyl, für Cyanmethyl, Cyanethyl oder für Benzyl steht,

$R^8$ für Wasserstoff, Methyl, Ethyl, n-oder i-Propyl, n-, i-, s-oder t-Butyl steht und

X für Sauerstoff, Schwefel, eine Sulfinylgruppe oder eine Sulfonylgruppe steht.

Ganz besonders bevorzugt sind Verbindungen der Formel (I), bei welchen

$R^1$ und $R^2$ gemeinsam mit den Kohlenstoffatomen, an welche sie gebunden sind für einen gegebenenfalls ein-bis dreifach durch Methyl substituierten Cyclohexenylenrest stehen,

$R^3$ und $R^4$ unabhängig voneinander jeweils für Fluor, Chlor oder Brom stehen,

$R^5$ für Wasserstoff, Methyl, Ethyl oder Phenyl steht,

$R^6$ für Wasserstoff oder Methyl steht,

$R^7$ für Wasserstoff, Methyl, Ethyl, n-oder i-Propyl, Allyl, Propargyl, Methoxymethyl, Methylthiomethyl, Methoxycarbonylmethyl, Ethoxycarbonylmethyl, Cyanmethyl, Cyanethyl oder Benzyl steht,

$R^8$ für Methyl oder Ethyl steht und

X für Sauerstoff, Schwefel, eine Sulfinylgruppe oder eine Sulfonylgruppe steht.

Im einzelnen seien außer den bei den Herstellungsbeispielen genannten Verbindungen, die folgenden N-Arylpyrrolin-2,5-dione der allgemeinen Formel (I) genannt:

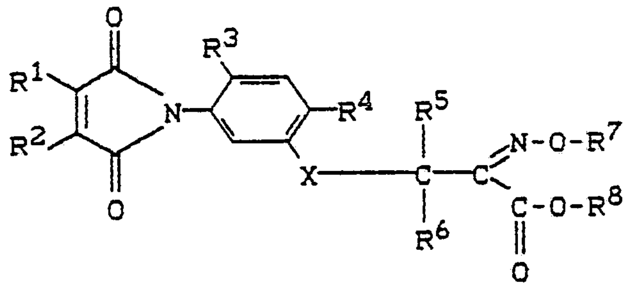

(I)

| 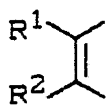 | $R^3$ | $R^4$ | $R^5$ | $R^6$ | $R^7$ | $R^8$ | X |
|---|---|---|---|---|---|---|---|
| 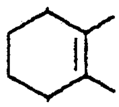 | F | Cl | $CH_3$ | H | $CH_3$ | $CH_3$ | O |
| 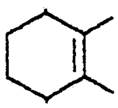 | F | Cl | $CH_3$ | H | $CH_3$ | $C_2H_5$ | O |
| 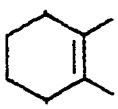 | F | Cl | $CH_3$ | H | $C_2H_5$ | $CH_3$ | O |
| 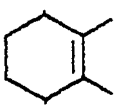 | F | Cl | $CH_3$ | H | $C_2H_5$ | $C_2H_5$ | O |
| 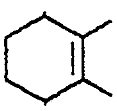 | F | Cl | H | H | $CH_3$ | $CH_3$ | S |

| 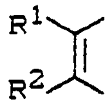 | $R^3$ | $R^4$ | $R^5$ | $R^6$ | $R^7$ | $R^8$ | X |
|---|---|---|---|---|---|---|---|
| 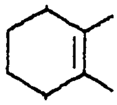 | F | Cl | H | H | $CH_3$ | $CH_3$ | S |
| 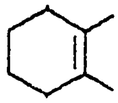 | F | Cl | H | H | $CH_3$ | $C_2H_5$ | S |
| 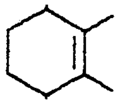 | F | Cl | H | H | $C_2H_5$ | $CH_3$ | S |
| 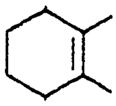 | F | Cl | H | H | $C_2H_5$ | $C_2H_5$ | S |
| 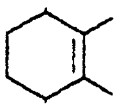 | F | Cl | H | H | $CH_3$ | $CH_3$ | SO |
| 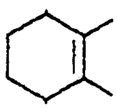 | F | Cl | H | H | $CH_3$ | $C_2H_5$ | SO |
| 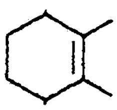 | F | Cl | H | H | $C_2H_5$ | $CH_3$ | SO |
| 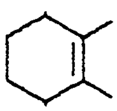 | F | Cl | H | H | $C_2H_5$ | $C_2H_5$ | SO |

| 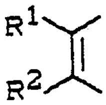 | $R^3$ | $R^4$ | $R^5$ | $R^6$ | $R^7$ | $R^8$ | X |
|---|---|---|---|---|---|---|---|
| 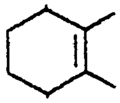 | F | Cl | H | H | $CH_3$ | $C_2H_5$ | $SO_2$ |
| 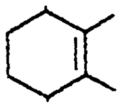 | F | Cl | H | H | $C_2H_5$ | $CH_3$ | $SO_2$ |
| 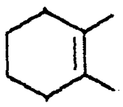 | F | Cl | H | H | $C_2H_5$ | $C_2H_5$ | $SO_2$ |
| 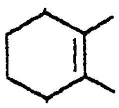 | F | Cl | $CH_3$ | H | $CH_3$ | $CH_3$ | S |
| 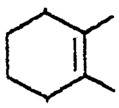 | F | Cl | $CH_3$ | H | $CH_3$ | $C_2H_5$ | S |
| 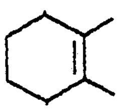 | F | Cl | $CH_3$ | H | $C_2H_5$ | $CH_3$ | S |
| 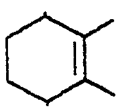 | F | Cl | $CH_3$ | H | $C_2H_5$ | $C_2H_5$ | S |
| 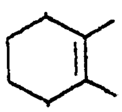 | F | Cl | $CH_3$ | H | $CH_3$ | $CH_3$ | SO |

| 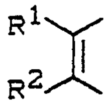 | $R^3$ | $R^4$ | $R^5$ | $R^6$ | $R^7$ | $R^8$ | X |
|---|---|---|---|---|---|---|---|
| 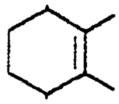 | F | Cl | $CH_3$ | H | $CH_3$ | $C_2H_5$ | SO |
| 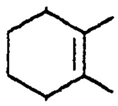 | F | Cl | $CH_3$ | H | $C_2H_5$ | $CH_3$ | SO |
| 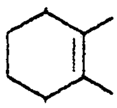 | F | Cl | $CH_3$ | H | $C_2H_5$ | $C_2H_5$ | SO |
| 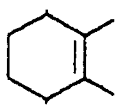 | F | Cl | $CH_3$ | H | $CH_3$ | $CH_3$ | $SO_2$ |
| 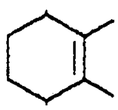 | F | Cl | $CH_3$ | H | $CH_3$ | $C_2H_5$ | $SO_2$ |
| 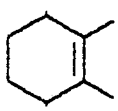 | F | Cl | $CH_3$ | H | $C_2H_5$ | $CH_3$ | $SO_2$ |
| 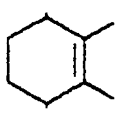 | F | Cl | $CH_3$ | H | $C_2H_5$ | $C_2H_5$ | $SO_2$ |
| 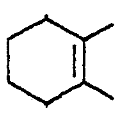 | Cl | Cl | H | H | $CH_3$ | $CH_3$ | O |

| 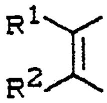 | $R^3$ | $R^4$ | $R^5$ | $R^6$ | $R^7$ | $R^8$ | X |
|---|---|---|---|---|---|---|---|
| 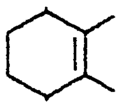 | Cl | Cl | H | H | $C_2H_5$ | $CH_3$ | O |
| 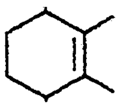 | Cl | Cl | H | H | $C_2H_5$ | $C_2H_5$ | O |
| 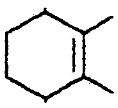 | Cl | Cl | $CH_3$ | H | $CH_3$ | $CH_3$ | O |
| 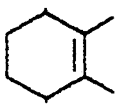 | Cl | Cl | $CH_3$ | H | $CH_3$ | $C_2H_5$ | O |
| 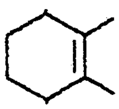 | Cl | Cl | $CH_3$ | H | $C_2H_5$ | $CH_3$ | O |
| 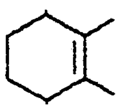 | Cl | Cl | $CH_3$ | H | $C_2H_5$ | $C_2H_5$ | O |
| 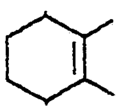 | Cl | Cl | H | H | $CH_3$ | $CH_3$ | S |
| 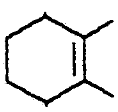 | Cl | Cl | H | H | $CH_3$ | $C_2H_5$ | S |

| 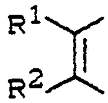 | $R^3$ | $R^4$ | $R^5$ | $R^6$ | $R^7$ | $R^8$ | X |
|---|---|---|---|---|---|---|---|
| 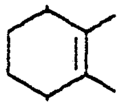 | Cl | Cl | H | H | $C_2H_5$ | $CH_3$ | S |
| 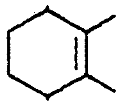 | Cl | Cl | H | H | $C_2H_5$ | $C_2H_5$ | S |
| 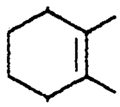 | Cl | Cl | H | H | $CH_3$ | $CH_3$ | SO |
| 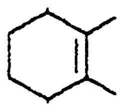 | Cl | Cl | H | H | $CH_3$ | $C_2H_5$ | SO |
| 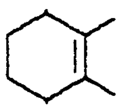 | Cl | Cl | H | H | $C_2H_5$ | $CH_3$ | SO |
| 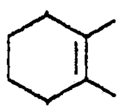 | Cl | Cl | H | H | $C_2H_5$ | $C_2H_5$ | SO |
| 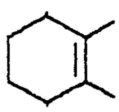 | Cl | Cl | H | H | $CH_3$ | $CH_3$ | $SO_2$ |
| 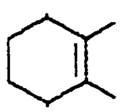 | Cl | Cl | H | H | $CH_3$ | $C_2H_5$ | $SO_2$ |

| 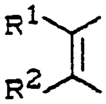 | $R^3$ | $R^4$ | $R^5$ | $R^6$ | $R^7$ | $R^8$ | X |
|---|---|---|---|---|---|---|---|
| 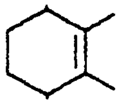 | Cl | Cl | H | H | $C_2H_5$ | $CH_3$ | $SO_2$ |
| 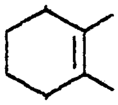 | Cl | Cl | H | H | $C_2H_5$ | $C_2H_5$ | $SO_2$ |
| 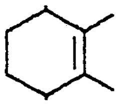 | Cl | Cl | $CH_3$ | H | $CH_3$ | $CH_3$ | S |
| 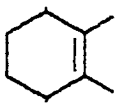 | Cl | Cl | $CH_3$ | H | $CH_3$ | $C_2H_5$ | S |
| 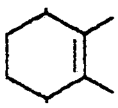 | Cl | Cl | $CH_3$ | H | $C_2H_5$ | $CH_3$ | S |
| 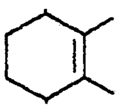 | Cl | Cl | $CH_3$ | H | $C_2H_5$ | $C_2H_5$ | S |
| 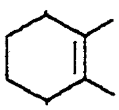 | Cl | Cl | $CH_3$ | H | $CH_3$ | $CH_3$ | SO |
| 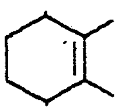 | Cl | Cl | $CH_3$ | H | $CH_3$ | $C_2H_5$ | SO |

| 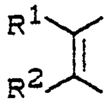 | $R^3$ | $R^4$ | $R^5$ | $R^6$ | $R^7$ | $R^8$ | X |
|---|---|---|---|---|---|---|---|
| 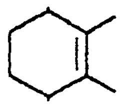 | Cl | Cl | $CH_3$ | H | $C_2H_5$ | $CH_3$ | SO |
| 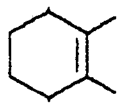 | Cl | Cl | $CH_3$ | H | $C_2H_5$ | $C_2H_5$ | SO |
| 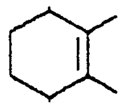 | Cl | Cl | $CH_3$ | H | $CH_3$ | $CH_3$ | $SO_2$ |
| 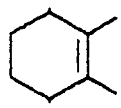 | Cl | Cl | $CH_3$ | H | $CH_3$ | $C_2H_5$ | $SO_2$ |
| 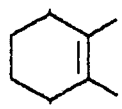 | Cl | Cl | $CH_3$ | H | $C_2H_5$ | $CH_3$ | $SO_2$ |
| 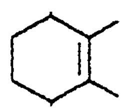 | Cl | Cl | $CH_3$ | H | $C_2H_5$ | $C_2H_5$ | $SO_2$ |

Verwendet man beispielsweise N-(4'-Chlor-2'-fluor-5'-hydroxy)-phenyl-3,4,5,6-tetrahydrophthalimid und Brombrenztraubensäuremethylester-O-methyloxim als Ausgangsverbindungen, so läßt sich der Reaktionsablauf des erfindungsgemäßen Verfahrens (a) durch das folgende Formelschema darstellen:

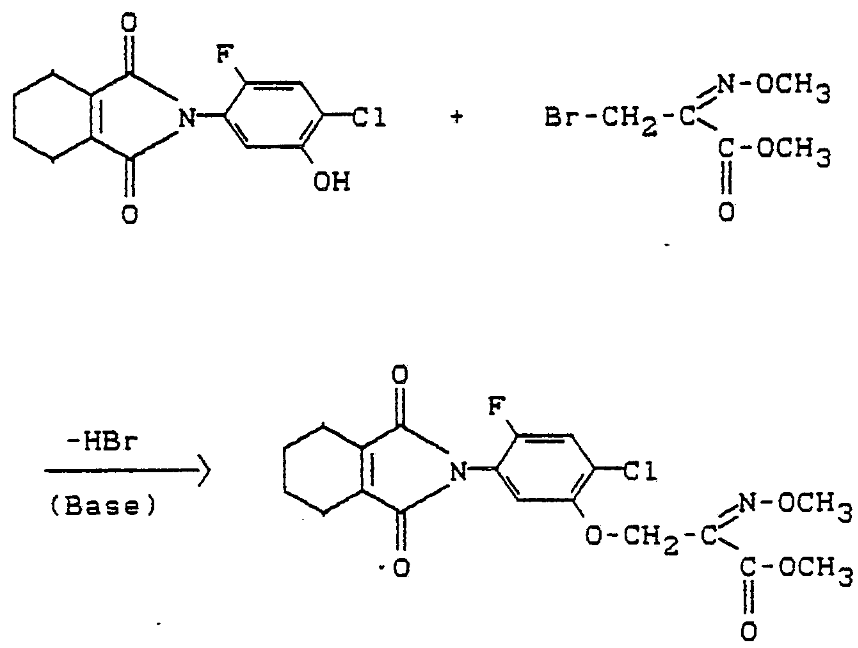


Verwendet man beispielsweise N-(4'-Chlor-2'-fluor-5'-(2-methoximino-2-ethoxycarbonyl-ethylthio)-phenyl]-3,4,5,6-tetrahydrophthalimid als Ausgangsverbindung und m-Chlorperbenzoesäure als Oxidationsmittel, so läßt sich der Reaktionsablauf des erfindungsgemäßen Verfahrens (b) durch das folgende Formelschema darstellen:

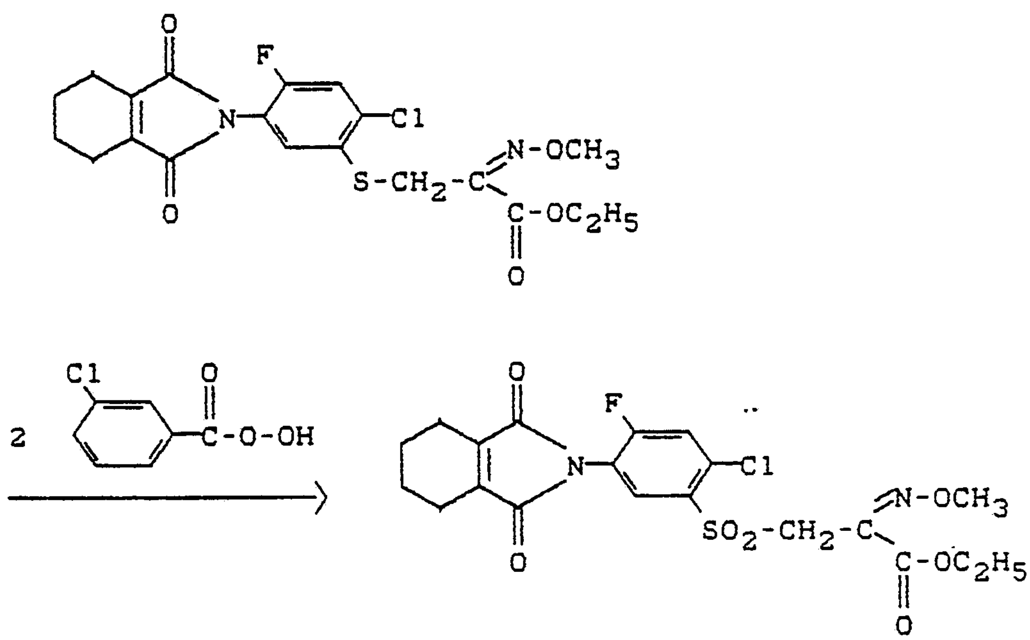


Verwendet man beispielsweise 3,4,5,6-Tetrahydrophthalsäureanhydrid und 2-Methoximino-3-(2,4-dichlor-5-aminophenoxy)-propionsäureethylester als Ausgangsverbindungen, so läßt sich der Reaktionsablauf des erfindungsgemäßen Verfahrens (c) durch das folgende Formelschema darstellen:

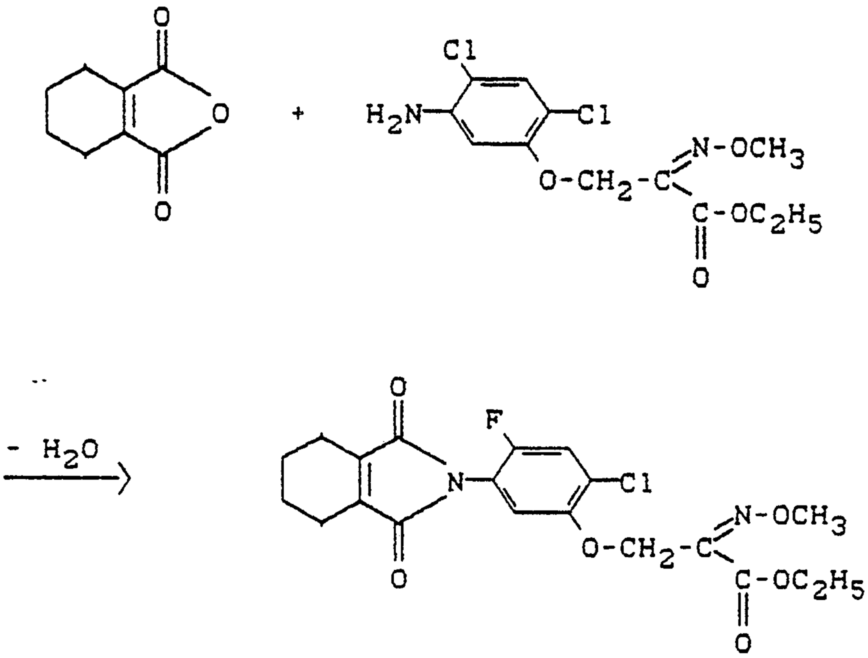

Die zur Durchführung des erfindungsgemäßen Verfahrens (a) als Ausgangsstoffe benötigen Pyrrolin-2.5-dione sind durch die Formel (II) allgemein definiert. In dieser Formel (II) stehen $R^1$, $R^2$, $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

$X^1$ steht vorzugsweise für Sauerstoff oder Schwefel.

Die Pyrrolin-2,5-dione der Formel (II) sind größtenteils bekannt (vgl. z.B. DE-OS 30 13 162 oder EP 61 741) oder erhältlich in Analogie zu bekannten Verfahren (vgl. auch EP 83 055 oder EP 95 192).

Die zur Durchführung des erfindungsgemäßen Verfahrens (a) weiterhin als Ausgangsstoffe benötigen α-Oximinocarbonsäureester sind durch die Formel (III) allgemein definiert. In dieser Formel (III) stehen $R^5$, $R^6$, $R^7$ und $R^8$ vorzugsweise für diejenigen Reste, die bereits im Zusammenhang mit der Beschreibung der erfindungsgemäßen Stoffe der Formel (I) als bevorzugt für diese Substituenten genannt wurden.

Y steht vorzugsweise für Halogen, insbesondere für Chlor oder Brom, oder für jeweils gegebenenfalls substituiertes Alkylsulfonyloxy oder Arylsulfonyloxy, wie beispielsweise Methansulfonyloxy oder p-Toluolsulfonyloxy.

Die α-Oximinocarbonsäureester der Formel (III) sind ebenfalls größenteils bekannt oder erhältlich in Analogie zu bekannten Verfahren (vgl. z.B. J. Chem. Soc. Chem. Commun. 1979, 1098; DE-OS 3 221 215, DE-OS 3 220 523, DE-OS 3 220 524 oder DE-OS 3 336 598).

Die zur Durchführung des erfindungsgemäßen Verfahrens (b) als Ausgangsstoffe benötigten N-Arylpyrrolin-2,5-dione sind durch die Formel (Ic) allgemein definiert. In dieser Formel (Ic) stehen $R^1$, $R^2$, $R^3$, $R^4$, $R^5$, $R^6$, $R^7$ und $R^8$ vorzugsweise für diejenigen Reste, die bereits im Zusammenhang mit der Beschreibung der erfindungsgemäßen Stoffe der Formel (I) als bevorzugt für diese Substituenten genannt wurden.

Die N-Arylpyrrolin-2,5-dione der Formel (Ic) sind erfindungsgemäße Verbindungen und erhältlich mit Hilfe der erfindungsgemäßen Verfahren (a) oder (c).

Die zur Durchführung des erfindungsgemäßen Verfahrens (c) als Ausgangsstoffe benötigen 1,2-Dicarbonsäureanhydride sind durch die Formel (IV) allgemein definiert. In dieser Formel (IV) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die 1,2-Dicarbonsäureanhydride der Formel (IV) sind allgemein bekannte Verbindungen der organischen Chemie oder erhältlich in Analogie zu allgemein bekannten Verfahren (vgl. z.B. Gazz. chim. ital. 57, 300-311 [1927]).

Die zur Durchführung des erfindungsgemäßen Verfahrens (c) weiterhin als Ausgangsstoffe benötigten Anilinderivate sind durch die Formel (V) allgemein definiert. In dieser Formel (V) stehen $R^3$, $R^4$, $R^5$, $R^6$, $R^7$, $R^8$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die Anilinderivate der Formel (V) sind noch nicht bekannt.

Man erhält sie, wenn man allgemein bekannte (Thio)Phenole der Formel (VI),

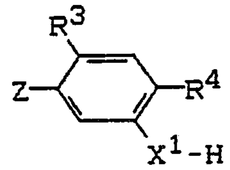

(VI)

in welcher
$R^3$ und $R^4$ die oben angegebene Bedeutung haben,
$X^1$ für Sauerstoff oder Schwefel steht und
Z für eine Amino-oder eine Nitrogruppe steht,
mit α-Oximinocarbonsäureestern der Formel (III),

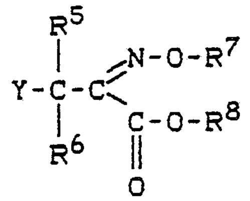

(III)

in welcher
$R^5$, $R^6$, $R^7$ und $R^8$ die oben angegebene Bedeutung haben und
Y für eine elektronenanziehende Abgangsgruppe, insbesondere für Halogen oder für jeweils gegebenenfalls substituiertes Alkylsulfonyloxy oder Arylsulfonyloxy steht,
in Analogie zur Durchführung des erfindungsgemäßen Verfahrens (a) gegebenenfalls in Gegenwart eines Verdünnungsmittels wie beispielsweise Acetonitril oder Essigsäureethylester und gegebenenfalls in Gegenwart eines Säurebindemittels wie beispielsweise Kaliumcarbonat oder Triethylamin bei Temperaturen zwischen 20 °C und 120 °C umsetzt, und im dem Fall, wo Z für eine Nitrogruppe steht, die so erhältichen Nitrophenyl(thio)ether der Formel (VII),

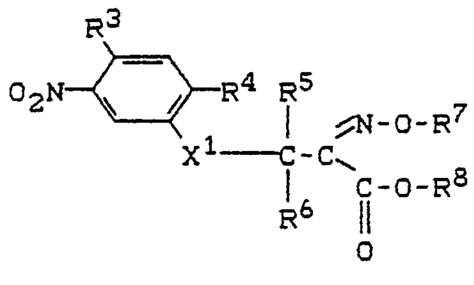

(VII)

in welcher
$R^3$, $R^4$, $R^5$, $R^6$, $R^7$, $R^8$ und $X^1$ die oben angegebene Bedeutung haben,
mit üblichen Reduktionsmitteln wie beispielsweise molekularem Wasserstoff, Zinndichlorid, Hydrazin oder Natriumsulfid gegebenenfalls in Gegenwart eines Katalysators in Analogie zu allgemein bekannten Verfahren an der Nitrogruppe reduziert (vgl. z. B. Organikum, VEB Deutscher Verlag der Wissenschaften, Berlin 1981, S. 645f);
Anilinderivate der Formel (Va),

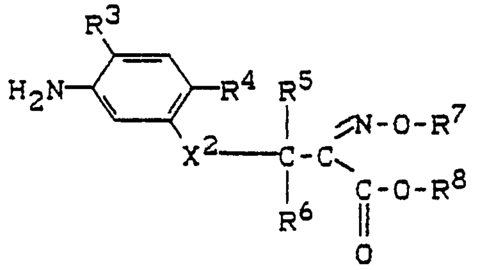

(Va)

in welcher
$R^3$, $R^4$, $R^5$, $R^6$, $R^7$ und $R^8$ die oben angegebene Bedeutung haben und
$X^2$ für eine Sulfinylgruppe oder für eine Sulfonylgruppe steht,
erhält man aus Anilinderivaten der Formel (Vb),

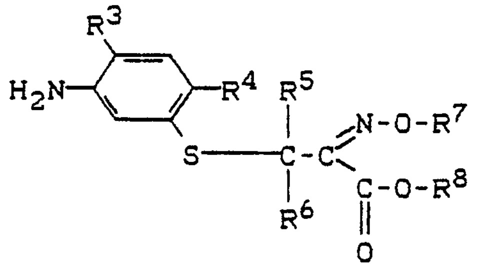

(Vb)

in welcher
$R^3$, $R^4$, $R^5$, $R^6$, $R^7$ und $R^8$ die oben angegebene Bedeutung haben.
indem man mit einem üblichen Oxidationmittel wie beispielsweise m-Chlorperbenzoesäure gegebenenfalls in Gegenwart eines Verdünnungsmittels wie beispielsweise Dichlormethan bei Temperaturen zwischen - 20 °C und + 70 °C in Analogie zur Durchführung des erfindungsgemäßen Verfahrens (b) umsetzt.

Als Verdünnungsmittels zur Durchführung des erfindungsgemäßen Verfahrens (a) kommen inerte organische Lösungsmittel infrage.

Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) wird vorzugsweise in Gegenwart eines geeigneten Säurebindemittels durchgeführt.

Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DDU).

Die Reaktionstemperaturen können bei der Durchführung des erfindungsgemäßen Verfahrens (a) in einem größeren Bereich variiert werden. Im allgemeinen arbeitet man bei Temperaturen zwischen - 20 °C und + 150 °C, vorzugsweise bei Temperaturen zwischen + 20 °C und +120 °C.

Zur Durchführung des erfindungsgemäßen Verfahrens (a) setzt man pro Mol an Pyrrolin-2,5-dion der Formel (II) im allgemeinen 1,0 bis 2,0 Mol, vorzugsweise 1,0 bis 1,2 Mol an $\alpha$-Oximinocarbonsäureester der Formel (III) und 1,0 bis 2,0 Mol, vorzugsweise 1,0 bis 1,5 Mol an Säurebindemittel ein. Die Reaktionsdurchführung, Aufarbeitung und Isolierung der Reaktionsprodukte der Formel (Ia) erfolgt nach üblichen Methoden in Analogie zu bekannten Verfahren (vgl. z.B. DE-OS 3 221 215 sowie die Herstellungsbeispiele).

Als Oxidationsmittel zur Durchführung des erfindungsgemäßen Verfahrens (b) kann man alle üblicherweise für Schwefeloxidationen infrage kommenden anorganischen oder organischen Oxidationsmittel verwenden. Vorzugsweise verwendet man organische Persäuren, wie beispielsweise Peressigsäure, 4-Nitroperbenzoesäure oder 3-Chlorperbenzoesäure, anorganische Persäuren, wie beispielsweise Periodsäure oder

auch Wasserstoffperoxid, Kaliumpermanganat oder Chromsäure.

Als Verdünnungsmittel zur Durchführung des erfindungsgemäßen Verfahren (b) kommen in Abhängigkeit vom verwendeten Oxidationsmittel organische Lösungsmittel oder wässrige Systeme infrage.

Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure; dipolar aprotische Lösungsmittel, wie Acetonitril, Aceton, Essigsäureethylester oder Dimethylformamid; Alkohole wie Methanol oder Ethanol oder deren Gemische mit Wasser.

Das erfindungsgemäße Verfahren (b) kann gegebenenfalls in Gegenwart eines Säurebindemittels durchgeführt werden. Als solche kommen alle üblicherweise verwendbaren organischen oder anorganischen Säurebindemittel in Frage. Vorzugsweise verwendet man Erdalkali-oder Alkalimetalhydroxide, -acetate oder -carbonate, wie beispielsweise Calciumhydroxid, Natriumhydroxid, Natriumacetat oder Natriumcarbonat.

Das erfindungsgemäße Verfahren (b) kann gegebenenfalls in Gegenwart eines geeigneten Katalysators durchgeführt werden. Als solche kommen alle üblicherweise für derartige Schwefeloxidationen üblichen Katalysatoren infrage. Vorzugsweise verwendet man Schwermetallsalze, wie beispielsweise Ammoniummolybdat.

Die Reaktionstemperaturen können bei der Durchführung des erfindungsgemäßen Verfahrens (b) in einem größeren Bereich variiert werden. Im allgemeinen arbeitet man bei Temperaturen zwischen - 20 °C und + 70 °C, vorzugsweise bei Temperaturen zwischen 0 °C und + 50 °C.

Zur Durchführung des erfindungsgemäßen Verfahrens (b) setzt man pro Mol an N-Arylpyrrolin-2,5-dion der Formel (Ic) im allgemeinen 0,8 bis 1,2 Mol, vorzugsweise äquimolare Mengen an Oxidationsmittel ein, wenn man die Oxidation des Schwefels auf der Sulfoxidstufe unterbrechen will. Will man den Schwefel bis zur Sulfonstufe oxidieren, setzt man pro Mol an N-Arylpyrrolin-2,5-dion der Formel (Ic) im allgemeinen 1,8 bis 3,0 Mol, vorzugsweise doppelt molare Mengen an Oxidationsmittel ein. Die Reaktionsdurchführung, Aufarbeitung und Isolierung der Reaktionsprodukte der Formel (Ib) erfolgt nach allgemein bekannten Methoden.

Als Verdünnungsmittel zur Durchführung des erfindungsgemäßen Verfahrens (c) kommen inerte organische Lösungsmittel infrage, Vorzugsweise verwendet man die bei Verfahren (a) genannten organischen Lösungsmittel oder Carbonsäuren wie Essigsäure oder Propionsäure.

Das erfindungsgemäße Verfahren (c) kann gegebenenfalls in Gegenwart eines geeigneten Reaktionshilfsmittels durchgeführt werden. Vorzugsweise verwendet man anorganische oder organische Säuren wie beispielsweise Essigsäure oder p-Toluolsulfonsäure, Anhydride wie beispielsweise Acetanhydrid oder Säurechloride wie Acetylchlorid als Reaktionshilfsmittel. Es ist auch möglich andere übliche wasserabspaltende Mittel wie beispielsweise N,N'-Dicyclohexylcarbodiimid als Reaktionshilfsmittel zu verwenden. Außerdem ist es auch möglich, die als Reaktionshilfsmittel verwendeten Säuren in entsprechendem Überschuß gleichzeitig als Verdünnungsmittel zu verwenden.

Die Reaktionstemperaturen können bei der Durchführung des erfindungsgemäßen Verfahrens (c) in einem größeren Bereich variiert werden. Im allgemeinen arbeitet man bei Temperaturen zwischen 20 °C und 180 °C, vorzugsweise bei Temperaturen zwischen 50 °C und 150 °C.

Zur Durchführung des erfindungsgemäßen Verfahrens (c) setzt man pro Mol an 1,2-Dicarbonsäureanhydrid der Formel (IV) im allgemeinen 0,8 bis 1,5 Mol, vorzugsweise 1,0 bis 1,2 Mol an Anilinderivat der Formel (V) und gegebenenfalls 0,01 bis 1,2 Mol an Reaktionshilfsmittel ein. Dabei ist es möglich, das erfindungsgemäße Verfahren (c) entweder in einer einstufigen Reaktionsführung durchzuführen oder alternativ die intermediär auftretenden Zwischenprodukte der Formel (VIII),

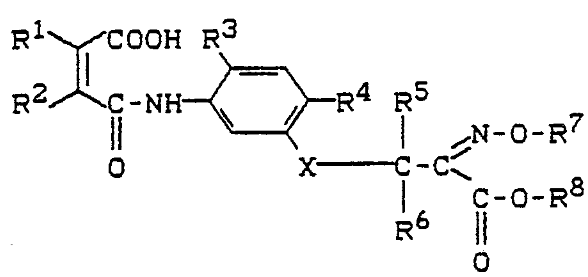

(VIII)

in welcher

$R^1$, $R^2$, $R^3$, $R^4$, $R^5$, $R^6$, $R^7$, $R^8$ und X die oben angegebene Bedeutung haben,

zu isolieren und in einer separaten 2, Reaktionsstufe zu cyclisieren.

Bei der einstufigen Reaktionsdurchführung setzt man das 1,2-Dicarbonsäureanhydrid der Formel (VI) und das Anilinderivat der Formel (V) direkt mit einer entsprechenden Menge an Reaktionshilfsmittel in einem geeigneten Verdünnungsmittel bei Temperaturen zwischen 50 °C und 180 °C um.

Bei der zweistufigen Reaktionsvariante arbeitet man in der ersten Reaktionsstufe ohne Reaktionshilfsmittel in einem inerten Verdünnungsmittel bei Temperaturen zwischen 20 °C und 80 °C und setzt die als Reaktionshilfsmittel verwendete Säure bzw. deren Derivate wie Anhydrid oder Säurechlorid oder auch andere wasserabspaltende Mittel wie beispielsweise N,N'-Dicyclohexylcarbodiimid erst in der 2. Reaktionsstufe zu, welche man zwischen 50 °C und 180 °C durchführt. Die Aufarbeitung und Isolierung der Zwischenprodukte der Formel (VIII) sowie der Endprodukte der Formel (I) erfolgt bei beiden Reaktionsvarianten nach allgemein üblichen und bekannten Methoden.

Die Zwischenprodukte der Formel (VIII) sind ebenfalls herbizid wirksam.

Die erfindungsgemäßen Wirkstoffe können als Defoliants, Desiccants, Krautabtötungsmittel und insbesondere als Unkrautvernichtungsmittel verwendet werden. Unter Unkraut im weitersten Sinne sind alle Pflanzen zu verstehen, die an Orten aufwachsen, wo sie unerwünscht sind, Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:
Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:
Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:
Echinochloa, Setaria, Panicum, Digitaria, Phleum, Poa, Festuca, Eleusine, Brachiaria, Lolium, Bromus, Avena, Cyperus, Sorghum, Argropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:
Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit and ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfen-anlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von dikotylen Unkräutern in mono-und dikotylen Kulturen, wie beispielsweise Weizen, Mais oder Soja einsetzten.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmittel, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenswasserstoffe, wie Chlorbenzole, Chlorethylene oder Melthylenchlorid, aliphatische Kohlenwasserstoffe, wie Cyclohexan oder Paraffine, z.B. Erdölfraktionen, Alkohole, wie Butanol oder Glycerol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder -schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangen, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Umkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethypropyl)-1,3,5-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Auch Mischungen mit 2-Chlor-N-{[(4-methoxy-6-methyl-1,3,5-triazin-2-yl)-amino]-carbonyl}-benzolsulfonamid;
N,N-Dimethyl-N'-(3-chlor-4-methylphenyl)-harnstoff;
N,N-Dimethyl-N'-(4-isopropylphenyl)-harnstoff;
4-Ethylamino-2-t-butylamino-6-methylthio-s-triazin;
2-Chlor-4-ethylamino-6-isopropylamino-1,3,5-triazin;
2-Chlor-4,6-bis-(ethylamino)-1,3,5-triazin;
2-Chlor-4-ethylamino-6-(3-cyanopropylamino)-1,3,5-triazin;

4-Amino-6-t-butyl-3-ethylthio-1,2,4-triazin-5-(4H)-on;
2-[4-(3,5-Dichlorpyrid-2-yloxy)-phenoxy]-propionsäure-(trimethylsilylmethylester);
2-[4-(2,4-Dichlorphenoxy)-phenoxy]-propionsäure, deren Methyl-oder deren Ethylester;
2-{4-[(6-Chlor-2-benzoxazolyl)-oxy]-phenoxy}-propansäure, deren Methyl-oder deren Ethylester;
Methyl-5-(2,4-dichlorphenoxy)-2-nitrobenzoat;
Methyl-2-[4,5-dihydro-4-methyl-4-(1-methylethyl)-5-oxo-1H-imidazol-2-yl]-4(5)-methylbenzoat;
3,5-Diiod-4-hydroxybenzonitril;
3,5-Dibrom-4-hydroxy-benzonitril;
2,4-Dichlorphenoxyessigsäure;
2,4-Dichlorphenoxypropionsäure;
(4-Chlor-2-methylphenoxy)-propionsäure;
(2-Methyl-4-chlorphenoxy)-essigsäure;
[(4-Amino-3,5-dichlor-6-fluor-2-pyridinyl)-oxy]-essigsäure bzw. deren 1-methylheptylester;
0-(6-Chlor-3-phenyl-pyridazin-4-yl)-S-octyl-thiocarbonat
oder 3-Isopropyl-2,1,3-benzothiadiazin-4-on-2,2-dioxid sind möglich.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1:

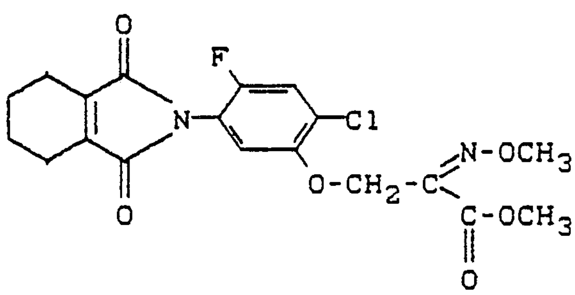

(Verfahren a)

Zu einer Suspension von 12,2 g (0,089 Mol) Kaliumcarbonat in 145 ml Acetonitril gibt man portionsweise 22 g (0,074 Mol) N-(4'-Chlor-2'-fluor-5'hydroxy)-phenyl-3,4,5,6-tetrahydrophthalimid, erhitzt nach beendeter Zugabe auf 70 °C und versetzt unter Rühren tropfenweise mit 20,2 g (0,079 Mol) Brombrenztraubensäuremethylester-O-methyloxim. Nach bendeter Zugabe rührt man weitere 12 Stunden bei 70 °C bis 75 °C, filtriert ausgefallenes Kaliumbromid ab und engt das Filtrat im Vakuum ein. Der

Rückstand wird in Toluol aufgenommen, mit Wasser gewaschen, über Natriumsulfat getrocknet und im Vakuum vom Lösungsmittel befreit. Das verbleibende Öl kristallisiert beim Verreiben mit Petrolether. Man erhält 26 g (82 % der Theorie) an N-[4'-Chlor-2'-fluor-5'-(2-methoximino-2-methoxycarbonylethyloxy)-phenyl]-3,4,5,6-tetrahydrophthalimid vom Schmelzpunkt 100 °C - 107 °C.

Herstellung der Ausgangsverbindungen

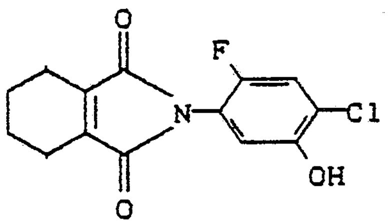

(II-1)

Eine Lösung von 37 g (0,23 Mol) 5-Amino-2-chlor-4-fluorphenol und 34,9 g (0,23 Mol) 3,4,5,6-Tetrahydrophthalsäureanhydrid in 100 ml Eisessig wird 16 Stunden unter Rückfluß erhitzt. Zur Aufarbeitung gibt man die Reaktionsmischung in Wasser und extrahiert mit Dichlormethan. Die Dichlormethanphase wird mehrmals mit Wasser gewaschen, über Natriumsulfat getrocknet und im Vakuum vom Lösungsmittel befreit.

Man erhält 34 g (50 % der Theorie) an N-(4'-Chlor-2'-fluor-5'-hydroxy-phenyl) - 3,4,5,6-tetrahydrophthalimid vom Schmelzpunkt 148 °C.

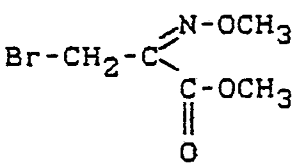

(III-1)

Zu einer Mischung aus 160 ml Chloroform und 73,1 g (0,87 Mol) O-Methylhydroxylaminhydrochlorid in 160 ml Wasser tropft man unter Rühren langsam 143 g (0,795 Mol) Brombrenztraubensäuremethylester. so daß die Temperatur 32 °C nicht übersteigt. Nach beendeter Zugabe rührt man weitere 4 Stunden bei 20 °C bis 25 °C, trennt dann die organische Phase ab, wäscht mehrmals mit Wasser, trocknet über Natriumsulfat und engt im Wasserstrahl-Vakuum ein. Der Rückstand wird im Hochvakuum destilliert. Man erhält 116 g (70 % der Theorie) an Brombenztraubensäuremethylester-O-methyloxim vom Siedepunkt 50 °C - 52 °C bei 0,25 mbar.

Beispiel 1 (alternative Herstellung)

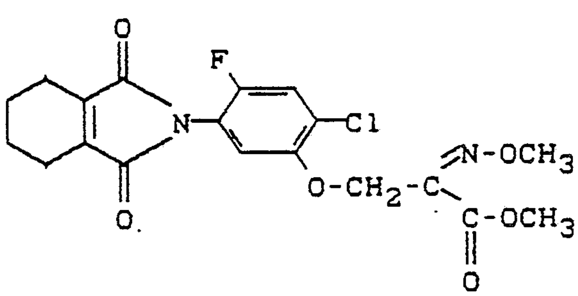

(Verfahren c)

11,6 g (0,04 Mol) 4-Chlor-2-fluor-5-(2-methoximino-2-methoxycarbonylethyloxy)-anilin und 6,2 g (0,04 mol) Cyclohexen-1,2-dicarbonsäureanhydrid in 20 ml Eisessig werden 7 Stunden unter Rückfluß erhitzt. Zur Aufarbeitung wird das Lösungsmittel im Vakuum abdestilliert. der Rückstand in Toluol aufgenommen, mit Wasser gewaschen, über Natriumsulfat getrocknet, im Vakumm eingeengt, mit Diisopropylether verrührt und abgesaugt.

Man erhält 11.1 g (65 % der Theorie) an N-[4'-Chlor-2'-fluor-5'-(2-methoximino-2-methoxycarbonylethyloxy)-phenyl]-3,4,5,6-tetrahydrophthalimid vom Schmelzpunkt 110 °C - 113 °C.

Herstellung der Ausgangsverbindungen:

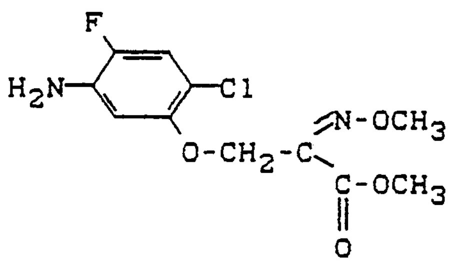

(V-1)

100 g (0,31 Mol) 3-(2-Chlor-4-fluor-5-nitrophenoxy)-2-methoximino-propionsäuremethylester werden in 500 ml Tetrahydrofuran gelöst und bei 18 °C bis 25 °C unter Zusatz von 10 g Raney-Nickel hydriert. Nach Beendigung der Wasserstoffaufnahme wird der Katalysator abfiltriert, das Filtrat im Vakuum eingeengt und der Rückstand aus Isopropanol umkristallisiert.

Man erhält 56 g (62 % der Theorie) an 4-Chlor-2-fluor-5-(2-methoximino-2-methoxycarbonylethyloxy)-anilin vom Schmelzpunkt 91 °C - 98 °C.

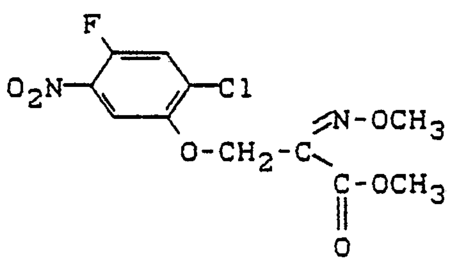

(VII-1)

Zu 76,6 g (0,4 Mol) 2-Chlor-4-fluor-5-nitrophenol (vgl. EP 61 741) und 63,5 g (0,46 Mol) Kaliumcarbonat gibt man bei 20 °C tropfenweise unter Rühren 96,6 g (0,46 Mol) Brombrenztraubensäuremethylester-O-methyloxim (vgl. DE-OS 3 220 523) und erhitzt nach beendeter Zugabe 4 Stunden auf Rückflußtemperatur. Zur Aufarbeitung wird das Lösungsmittel im Vakuum abdestilliert, der Rückstand in Dichlormethan aufgenommen, mit Wasser gewaschen, über Natriumsulfat getrocknet und im Vakuum eingeengt.

Man erhält 129 g (100 % der Theorie) an 3-(2-Chlor-4-fluor-5-nitrophenoxy)-2-methoximino-propionsäuremethylester vom Schmelzpunkt 67 °C - 72 °C.

In entsprechender Weise und gemäß den allgemeinen Angaben zur Herstellung erhält man die folgenden N-Arylpyrrolin-2,5-dione der allgemeinen Formel (I):

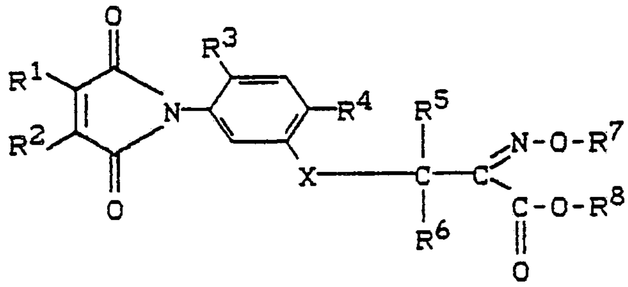

(I)

| Bsp Nr. | 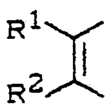 | $R^3$ | $R^4$ | $R^5$ | $R^6$ | $R^7$ | $R^8$ | X | physik. Eigenschaften |
|---|---|---|---|---|---|---|---|---|---|
| 2 | 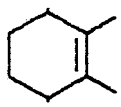 | F | Cl | H | H | $CH_3$ | $C_2H_5$ | O | Fp 86-89 °C |
| 3 | 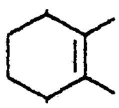 | Cl | Cl | H | H | $CH_3$ | $C_2H_5$ | O | $^1$H-NMR*): 1,32(t;3H) 4,03(s;3H) 4,86(s;2H) |
| 4 | 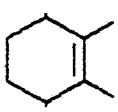 | F | Cl | H | H | $C_2H_5$ | $CH_3$ | O | $^1$H-NMR*): 1,3 (t;3H) 3,88(s;3H) 4,96(s;2H) |
| 5 | 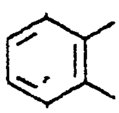 | F | Cl | H | H | $C_2H_5$ | $C_2H_5$ | O | $^1$H-NMR*): 1.32(t;3H) 1,37(t;3H) 4,96(s;2H) |
| 6 | 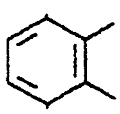 | F | Cl | H | H | $CH_3$ | $CH_3$ | O | Fp 134-137 °C |

| Bsp Nr. | 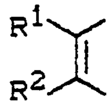 | $R^3$ | $R^4$ | $R^5$ | $R^6$ | $R^7$ | $R^8$ | X | physik. Eigenschaften |
|---|---|---|---|---|---|---|---|---|---|
| 7 | 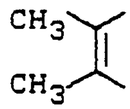 | F | Cl | H | H | $CH_3$ | $CH_3$ | O | Fp 93-95° C |
| 8 | 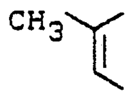 | F | Cl | H | H | $CH_3$ | $CH_3$ | O | Fp 80-81° C |
| 9 | 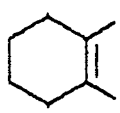 | H | Cl | H | H | $CH_3$ | $CH_3$ | S | zähes Öl |
| 10 | 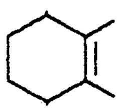 | H | Cl | H | H | $CH_3$ | $CH_3$ | $SO_2$ | Fp 70-73° C |
| 11 | 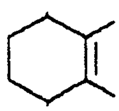 | F | $CH_3$ | H | H | $CH_3$ | $C_2H_5$ | O | Fp 102-104° C |

*) Die $^1$H-NMR-Spektren wurden in Deuterochloroform ($CDCl_3$) mit Tetramethylsilan (TMS) als innerem Standard aufgenommen. Angegebene ist die chemische Verschiebung als δ-Wert in ppm.

Anwendungsbeispiele:

In den folgenden Anwendungsbeispielen wurde die nachstehend aufgeführte Verbindung als Vergleichsubstanz eingesetzt:

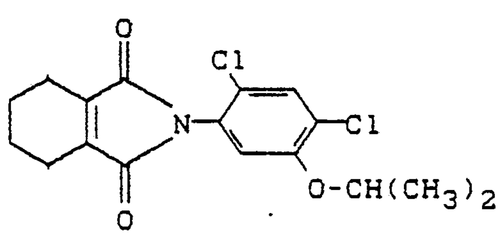

(A)

N-(2,4-Dichlor-5-isopropoxy-phenyl)-Δ'-tetrahydrophthalimid
(bekannt aus DE-OS 3 313 162)

Beispiel A

Pre-emergence-Test
Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten

0 % = keine Wirkung (wie unbehandelte Kontrolle)
100 % = totale Vernichtung

Im diesem Test zeigen beispielsweise die Verbindungen gemäß der Herstellungsbeispiele 1 und 2 eine deutliche Überlegenheit in der Wirksamkeit gegenüber der Vergleichssubstanz (A).

Beispiel B

Post-emergence-Test
Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wir der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigen beispielsweise die Verbindungen gemäß der Herstellungsbeispiele 1 und 2 eine deutliche Überlengenheit in der Wirksamkeit gegenüber der Vergleichssubstanz (A).

Beispiel C

Entlaubung und Austrocknung der Blätter bei Baumwolle
Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden in Gewächshaus bis zur vollen Entfaltung des 5. Falgeblattes angezogen. In diesem Stadium werden die Pflanzen tropfnaß mit den Wirkstoffzubereitungen besprüht. Nach 1 Woche

werden der Blattfall und das Austrocknen der Blätter im Vergleich zu den Kontrollpflanzen bonitiert. Es bedeuten:

0 kein Austrocknen der Blätter, kein Blattfall

\+ leichtes Austrocknen der Blätter, geringer Blattfall

\+ + starkes Austrocknen der Blätter, starker Blattfall

\+ + + sehr starkes Austrocknen der Blätter, sehr starker Blattfall

In diesem Test zeigen beispielsweise die Verbindungen gemäß der Herstellungsbeispiele 1, 2, 3 und 4 eine sehr gute Wirkung.

## Ansprüche

1. N-Arylpyrrolin-2,5-dione der allgemeinen Formel (I)

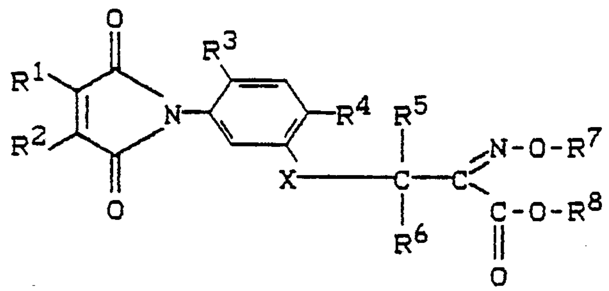

(I)

in welcher

$R^1$ und $R^2$ unabhängig voneinander jeweils für Wasserstoff, Alkyl, Halogenalkyl, Alkoxyalkyl, Alkoxycarbonylalkyl oder für gegebenenfalls substituiertes Aryl stehen, oder gemeinsam mit den Kohlenstoffatomen, an welche sie gebunden sind, für einen gegebenenfalls substituierten Cyclohexenylenrest oder für einen gegebenenfalls substituierten Phenylenrest stehen,

$R^3$ und $R^4$ unabhängig voneinander jeweils für Wasserstoff, Halogen, Alkyl, Alkoxy oder Aklylthio stehen,

$R^5$ Wasserstoff, Alkyl oder für gegebenenfalls substituiertes Aryl steht,

$R^6$ für Wasserstoff oder Alkyl steht,

$R^7$ für Wasserstoff, Alkly, Alkenyl, Alkinyl, Halogenalkyl, Halogenalkenyl, Alkoxyalkyl, Alkylthioalkyl, Alkoxycarbonylalkyl, Cyanankyl, Cycloalkyl, Cycloalkylalkyl oder für gegebenenfalls substituiertes Aralkyl steht,

$R^8$ für Wasserstoff oder Alkyl steht und

X für Saurerstoff, Schwefel, eine Sulfinylgruppe oder eine Sulfonylgruppe steht.

2. Verbindungen der allgemeinen Formel (I) gemäß Anspruch 1, bei welcehn

$R^1$ und $R^2$ unabhängig voneinander jeweils für Wasserstoff, für jeweils geradkettiges odr verzweigtes Alkyl, Alkoxyalkyl oder Alkoxycarbonylalkyl mit jeweils 1 bis 4 Kohlenstoffatomen in den einzelnen Alkylteilen, für geradkettiges oder verzweigtes Halogenalkyl mit 1 bis 4 Kohlenstoffatomen und 1 bis 9 gleichen oder berschiedenen Halogenatomen oder für gegenbenenfalls einfach oder mehrfach, gleich oder verschieden durch Halogen, Cyano, Nitro sowie jeweils geradkettiges oder verzweigtes Alkyl, Alkoxy, Alkylthio oder Halogenalkyl (mit jeweils 1 bis 4 Kohlenstoffatomen und im Fall des Halogenalkyl mit 1 bis 9 gleichen oder verschiedenen Halogenatomen) substituiertes Aryl mit 6 bis 10 Kohlenstoffatomen stehen, oder

$R^1$ und $R^2$ gemeinsam mit den Kohlenstoffatomen, an welche sie gebunden sind, für einen gegebenenfalls einfach oder mehrfach, gleich oder verschieden durch Alkyl mit 1 bis 4 Kohlenstoffatomen substituierten Cyclohexenylenrest oder für einen gegebenenfalls einfach oder mehrfach, gleich oder verschieden durch Halogen, Cyano, Nitro sowie jeweils geradkettiges oder verzweigtes Alkyl, Alkoxy oder Alkoxycarbonyl mit jeweils 1 bis 4 Kohlenstoffatomen sowie Halogenalkyl mit 1 bis 4 Kohlenstoffatomen und 1 bis 9 gleichen oder verschiedenen Halogenatomen, substituierten Phenylenrest stehen,

$R^3$ und $R^4$ unabhängig voneinander jeweils für Wasserstoff, Fluor, Chlor, Brom, Iod sowie jeweils geradkettiges oder verzweigtes Alkyl, Alkoxy oder Alkylthio mit jeweils 1 bis 4 Kohlenstoffatomen stehen,

$R^5$ für Wasserstoff, für geradkettiges oder verzweigtes Alkyl mit 1 bis 4 Kohlenstoffatomen oder für gegebenenfalls einfach oder mehrfach, gleich oder verschieden durch Fluor, Chlor, Brom, Iod, Cyano, Nitro sowie jeweils geradkettiges oder verzweigtes Alkyl oder Alkoxy mit jeweils 1 bis 4 Kohlenstoffatomen substituiertes Aryl mit 6 bis 10 Kohlenstoffatomen steht,

$R^6$ für Wasserstoff oder für jeweils geradkettiges oder verzweigtes Alkyl mit 1 bis 4 Kohlenstoffatomen steht,

$R^7$ für Wasserstoff, für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen, für jeweils geradkettiges oder verzweigtes Halogenalkyl mit 1 bis 6 Kohlenstoffatomen oder Halogenalkenyl mit 3 bis 6 Kohlenstoffatomen und jeweils 1 bis 9 gleichen oder verschiedenen Halogenatomen, für jeweils geradkettiges oder verzweigtes Alkoxyalkyl, Alkylthioalkyl, Alkoxycarbonylalkyl oder Cyanalkyl mit jeweils 1 bis 4 Kohlenstoffatomen in den einzelen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 4 Kohlenstoffatomen im geradkettigen oder verzweigten Alkylteil oder für gegebenenfalls im Arylteil einfach oder mehrfach, gleich oder verschieden durch Halogen, Cyano, Nitro, sowie jeweils geradkettiges oder verzweigtes Alkyl, Alkoxy oder Alkylthio mit jeweils 1 bis 4 Kohlenstoffatomen substituiertes, im Alkylteil geradkettiges oder verzweigtes Aralky mit 1 bis 4 Kohlenstoffatomen im Alkylteil und 6 bis 10 Kohlenstoffatomen im Arylteil steht,

$R^8$ für Wasserstoff oder für geradkettiges oder verzweigtes Alkyl mit 1 bis 4 Kohlenstoffatomen steht und

X für Sauerstoff, Schwefel, eine Sulfinylgruppe oder eine Sulfonylgruppe steht.

3. Verbindungen der allgemeinen Formel (I) gemäß Anspruch 1, in welcher:

$R^1$ und $R^2$ unabhängig voneinander jeweils für Wasserstoff, Methyl, Ethyl, Isopropyl, Chlormethyl, Trifluormethyl, Methoxymethyl oder Methoxycarbonylmethyl stehen oder gemeinsam mit den Kohlenstoffatomen, an welche sie gebunden sind, für einen gegenbenenfalls ein-bis dreifach durch Methyl substitutierten Cyclohexenylenrest oder für einen gegebenenfalls ein-bis vierfach, gleich oder verschieden durch Chlor, Nitro, Methyl und/oder Trifluormethyl substituierten Phenylenrest stehen,

$R^3$ und $R^4$ unabhängig voneinander jeweils für Wasserstoff, Fluor, Chlor, Brom, Methyl, Methyoxy oder Methylthio stehen,

$R^5$ für Wasserstoff, Methyl, Ethyl, n-oder i-Propyl, n-, i-, s-oder t-Butyl oder für Phenyl steht,

$R^6$ für Wasserstoff, Methyl oder Ethyl steht,

$R^7$ für Wasserstoff, Methyl, Ethyl, n-oder i-Propyl, n-, i-, s-oder t-Butyl, Allyl, n-oder i-Butenyl, Propargyl, n-oder i-Butinyl, für Chlorethyl, Trifluorethyl, Chlorallyl, Chlorbutenyl, Dichlorallyl, Cyclopropyl, Cyclopentyl, Cyclohexyl, Cyclopropymethyl, Cyclopentylmethyly, Cyclohexylmethyl, für Methoxymethyl, Methoxyethyl, Ethoxymethyl, Ethoxyethyl, Methylthiomethyl, Methylthioethyl, Methoxycarbonylmethyl, Ethoxycarbonylmethyl, Methoxycarbonylethyl, Ethoxycarbonylethyl, für Cyanmethyl, Cyanethyl oder für Benzyl steht,

$R^8$ für Wasserstoff, Methyl, Ethyl, n-oder i-Propyl, n-, i-, s-oder t-Butyl steht und

X für Sauerstoff, Schwefel, eine Sulfinylgruppe oder eine Sulfonylgruppe steht.

4. Verbindungen der allgemeinen Formel (I) gemäß Anspruch 1, bei welchen

$R^1$ und $R^2$ gemeinsam mit den Kohlenstoffatomen, an welche sie gebunden sind für einen gegebenenfalls ein-bis dreifach durch Methyl substituierten Cyclohexenylenrest stehen,

$R^3$ und $R^4$ unabhängig voneinander jeweils für Fluor, Chlor oder Brom stehen,

$R^5$ für Wasserstoff, Methyl, Ethyl oder Phenyl steht,

$R^6$ für Wasserstoff oder Methyl steht,

$R^7$ für Wasserstoff, Methyl, Ethyl, n-oder i-Propyl, Allyl, Propargyl, Methoxymethyl, Methylthiomethyl, Methoxycarbonylmethyl, Ethoxycarbonylmethy, Cyanmethyl, Cyanethyl oder Benzyl steht,

$R^8$ für Methyl oder Ethyl steht und

X für Sauerstoff, Schwefel, eine Sulfinylgruppe oder eine Sulfonylgruppe steht.

5. Verfahren zur Herstellung von N-Arylpyrrolin-2,5-dionen der allgemeinen Formel (I),

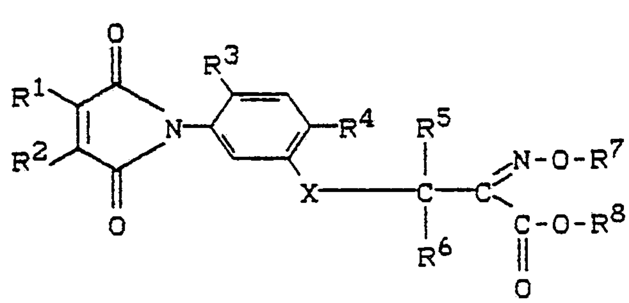

(I)

in welcher

$R^1$ und $R^2$ unabhängig voneinander jeweils für Wasserstoff, Alkyl, Halogenalkyl, Alkoxyalkyl, Alkoxycarbonylalkyl oder für gegebenenfalls substituiertes Aryl stehen, oder gemeinsam mit den Kohlenstoffatomen, an welche sie gebunden sind, für einen gegebenenfalls substituierten Cyclohexenylenrest oder für einen gegebenenfalls substituierten Phenylenrest stehen,

$R^3$ und $R^4$ unabhängig voneinander jeweils für Wasserstoff, Halogen, Alkyl, Alkoxy oder Alkylthio stehen,
$R^5$ für Wasserstoff, Alkyl oder für gegebenenfalls substituiertes Aryl steht,
$R^6$ für Wasserstoff oder Alkyl steht,
$R^7$ für Wasserstoff, Alkyl, Alkenyl, Alkinyl, Halogenalkyl, Halogenalkenyl, Alkoxyalkyl, Alkylthioalkyl, Alkoxycarbonylalkyl, Cyanalkyl, Cycloalkyl, Cycloalkylalkyl oder für gegebenenfalls substituiertes Aralkyl steht,
$R^8$ für Wasserstoff oder Alkyl steht und
X für Sauerstoff, Schwefel, eine Sulfinylgruppe oder eine Sulfonylgruppe steht,
dadurch gekennzeichnet, daß man

a) um N-Arylpyrrolin-2,5-dione der allgemeinen Formel (Ia)

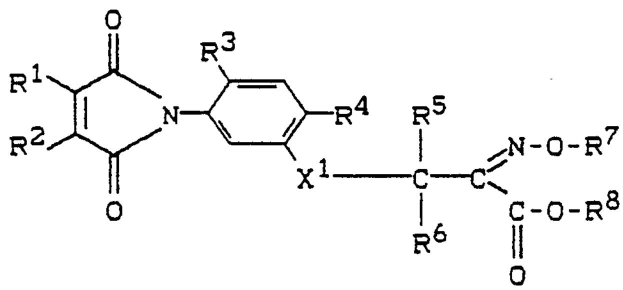

(Ia)

zu erhalten,
in welcher
$R^1$, $R^2$, $R^3$, $R^4$, $R^5$, $R^6$, $R^7$ und $R^8$ die oben angegebene Bedeutung haben und
$X^1$ für Sauerstoff oder Schwefel steht,
Pyrrolin-2,5-dione der allgemeinen Formel (II),

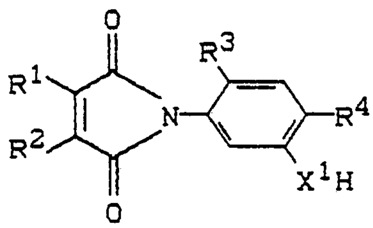

(II)

in welcher
$R^1$, $R^2$, $R^3$, $R^4$ und $X^1$ die oben angegebene Bedeutung haben,
mit α-Oximino-carbonsäurestern der Formel (III),

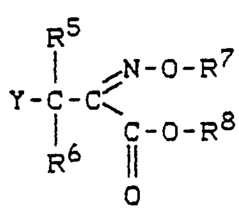

(III)

in welcher
$R^5$, $R^6$, $R^7$ und $R^8$ die oben angegebene Bedeutung haben und
Y für eine elektronenanziehende Abgangsgruppe steht,
gegebenenfalls in Gegenwart eines Verdünnungsmittels und gegebenenfalls in Gegenwart eines Säurebindemittels umsetzt; oder daß man,

b) um N-Arylpyrrolin-2,5-dione der allgemeinen Formel (Ib),

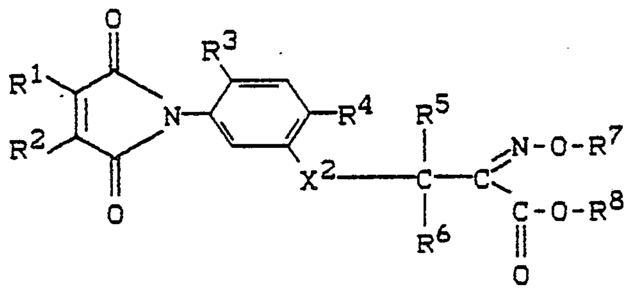

(Ib)

zu erhalten,
in welcher
$R^1$, $R^2$, $R^3$, $R^4$, $R^5$, $R^6$, $R^7$ und $R^8$ die oben angegebene Bedeutung haben und
$X^2$ für eine Sulfinyl oder eine Sulfonylgruppe steht,
die nach Verfahren (a) oder (c) erhältlichen N-Arylpyrrolin-2,5-dione der allgemeinen Formel (Ic),

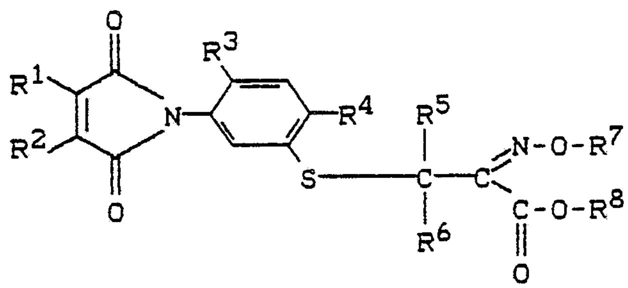

(Ic)

in welcher
$R^1$, $R^2$, $R^3$, $R^4$, $R^5$, $R^6$, $R^7$ und $R^8$ die oben angegebene Bedeutung haben,
mit einem Oxidationsmittel gegebenenfalls in Gegenwart eines Verdünnungsmittels, gegebenenfalls in Gegenwart eines Säurebindemittels und gegebenenfalls in Gegenwart eines Katalysators umsetzt; oder daß man,

c) um N-Arylpyrrolidin-2,5-dione der allgemeinen Formel (I)

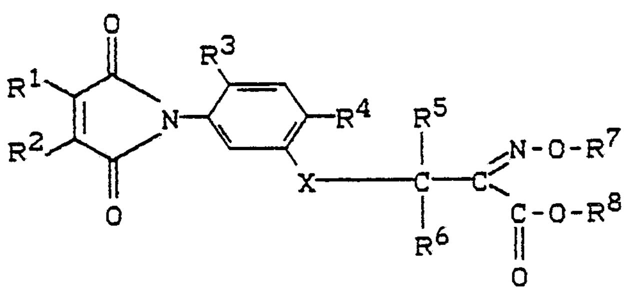

(I)

in welcher
$R^1$, $R^2$, $R^3$, $R^4$, $R^5$, $R^6$, $R^7$, $R^8$ und X die oben angegebene Bedeutung haben,
zu erhalten, alternativ auch 1,2-Dicarbonsäureanhydride der allgemeinen Formel (IV),

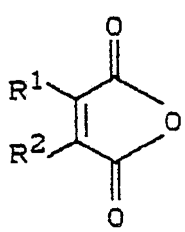

(IV)

in welcher

$R^1$ und $R^2$ die oben angegebene Bedeutung haben, mit Anilinderivaten der allgemeinen Formel (V),

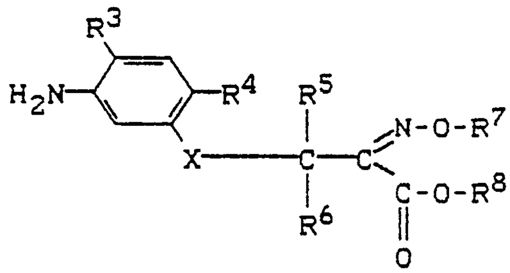

(V)

in welcher
$R^3$, $R^4$, $R^5$, $R^6$, $R^7$, $R^8$ und X die oben angegebene Bedeutung haben, gegebenenfalls in Gegenwart eines Verdünnungsmittels und gegebenenfalls in Gegenwart eines Reaktionshilfsmittels umsetzt.

6. Herbizide und pflanzenwachstumsregulierende Mittel, gekennzeichnet durch einen Gehalt an mindestens einem N-Arylpyrrolin-2,5-dion der allgemeinen Formel (I) gemäß den Ansprüchen 1 bis 5.

7. Verfahren zur Bekämpfung von Unkräutern und zur Regulierung des Pflanzenwuchses, dadurch gekennzeichnet, daß man N-Arylpyrrolin-2,5-dione der allgemeinen Formel (I) gemäß den Ansprüchen 1 bis 5 auf die Unkräuter und/oder ihren Lebensraum einwirken läßt.

8. Verwendung von N-Arylpyrrolin-2,5-dionen der allgemeinen Formel (I) gemäß den Ansprüchen 1 bis 5 zur Bekämpfung von Unkräutern und zur Regulierung des Pflanzenwuchses.

9. Verfahren zur Herstellung von herbiziden und pflanzenwachstumsregulierenden Mitteln, dadurch gekennzeichnet, daß man N-Arylpyrrolin-2,5-dione der allgemeinen Formel (I) gemäß den Ansprüchen 1 bis 5 mit Streckmitteln und/oder oberflächenaktiven Substanzen vermischt.

10. Anilinderivate der allgemeinen Formel (V),

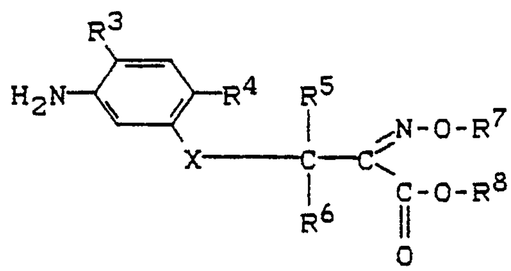

(V)

in welcher
$R^3$ und $R^4$ unabhängig voneinander jeweils für Wasserstoff, Halogen, Alkyl, Alkoxy oder Alkylthio stehen,
$R^5$ für Wasserstoff, Alkyl oder für gegebenenfalls substituiertes Aryl steht,
$R^6$ für Wasserstoff oder Alkyl steht,
$R^7$ für Wasserstoff, Alkyl, Alkenyl, Alkinyl, Halogenalkyl, Halogenalkenyl, Alkoxyalkyl, Alkylthioalkyl, Alkoxycarbonylalkyl, Cyanalkyl, Cycloalkyl, Cycloalkylalkyl oder für gegebenenfalls substituiertes Aralkyl steht,
$R^8$ für Wasserstoff oder Alkyl steht und
X für Sauerstoff, Schwefel, eine Sulfinylgruppe oder eine Sulfonylgruppe steht.

11. Verbindungen der allgemeinen Formel (VII)

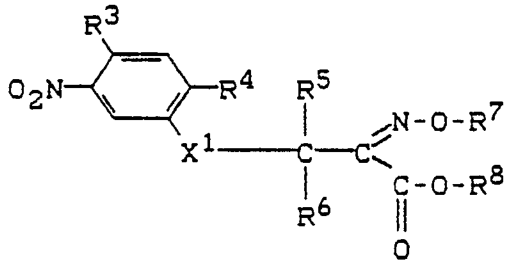

(VII)

in welcher
$R^3$ und $R^4$ unabhängig voneinander jeweils für Wasserstoff, Halogen, Alkyl, Alkoxy oder Alkylthio stehen,
$R^5$ für Wasserstoff, Alkyl oder für gegebenenfalls substituiertes Aryl steht,
$R^6$ für Wasserstoff oder Alkyl steht,
$R^7$ für Wasserstoff, Alkyl, Alkenyl, Alkinyl, Halogenalkyl, Halogenalkenyl, Alkoxyalkyl, Alkylthioalkyl, Alkoxycarbonylalkyl, Cyanalkyl, Cycloalkyl, Cycloalkylalkyl oder für gegebenenfalls substituiertes Aralkyl steht,
$R^8$ für Wasserstoff oder Alkyl steht und
X' für Sauerstoff oder Schwefel steht.

Europäisches Patentamt

# EUROPÄISCHER RECHERCHENBERICHT

Nummer der Anmeldung

EP 88105492.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2 - 0 190 755 (ASAHI KASEI KOGYO KABUSHIKI KAISHA)<br>* Patentansprüche 1,8,15 * | 1,6,7 | C 07 D 207/452<br>C 07 D 209/48<br>A 01 N 43/36<br>C 07 C 131/00<br>C 07 C 147/06<br>C 07 C 147/14<br>C 07 C 149/32 |
| A | US - A - 4 289 699 (MASAYUKI OBA et al.)<br>* Zusammenfassung * | 1 | |
| A | GB - A - 924 730 (UNITED STATES RUBBER COMPANY)<br>* Spalte 1 * | 1 | |
| A | US - A - 4 193 927 (MARCUS BAUMANN et al.)<br>* Spalten 1-4 * | 1 | RECHERCHIERTE SACHGEBIETE (Int. Cl.4) |
| D,A | DE - A1 - 3 013 162 (MITSUBISHI CHEMICAL INDUSTRIES, LTD.)<br>* Seiten 1-6 * | 1,6,7 | C 07 D 207/00<br>C 07 D 209/00<br>C 07 C 131/00<br>C 07 C 147/00<br>C 07 C 149/00 |
| A | DE - A - 2 248 941 (CHEMISCHE FABRIK VON HYDEN GMBH)<br>* Patentanspruch 1 * | 10,11 | |
| A | DE - A1 - 3 336 598 (BAYER AG)<br>* Zusammenfassung * | 10,11 | |

Der vorliegende Recherchenbericht wurde für alle Patentansprüche erstellt.

| Recherchenort | Abschlußdatum der Recherche | Prüfer |
|---|---|---|
| WIEN | 04-07-1988 | HEIN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82